# EUROPEAN PATENT SPECIFICATION

(11) **EP 3 375 291 B1**
(45) Date of publication and mention of the grant of the patent: **10.08.2022**
(21) Application number: 17161671.7
(22) Date of filing: 17.03.2017
(51) Int. Cl.: C12P 19/00, C12P 19/18, C12P 19/26, A23L 33/21, A23C 7/04, A23C 9/154, A23C 9/15, A23C 9/20, C07H 1/08, C07H 1/04, C07H 3/06, A23C 9/156, A61K 31/7016, C07H 3/04, C13B 50/00, C13K 5/00, C13K 7/00, C13K 13/00, A23L 29/30, A23L 33/00

(54) **A METHOD OF INHIBITING ISOMERIZATION OF A REDUCING SACCHARIDE UPON THERMAL TREATMENT**
VERFAHREN ZUR HEMMUNG DER ISOMERISIERUNG EINES REDUZIERENDEN SACCHARIDS NACH THERMISCHER BEHANDLUNG
PROCÉDÉ D'INHIBITION DE L'ISOMÉRISATION D'UN SACCHARIDE RÉDUCTEUR LORS DE TRAITEMENT THERMIQUE

(43) Date of publication of application: 19.09.2018
(73) Proprietor: Chr. Hansen HMO GmbH, 53619 Rheinbreitbach (DE)
(72) Inventor: Wartenberg, Dr. Dirk, 53225 Bonn (DE)
(74) Representative: Witte, Weller & Partner Patentanwälte mbB

(56) References cited:
- EP-A1- 0 349 712
- EP-A1- 3 315 610
- WO-A1-2015/032413
- WO-A1-2015/150328
- GB-A- 423 063
- US-A- 2 826 502
- US-A1- 2016 376 616
- MONTILLA A ET AL: "Egg shell as catalyst of lactose isomerisation to lactulose", FOOD CHEMISTRY, ELSEVIER LTD, NL, vol. 90, no. 4, 1 May 2005 (2005-05-01), pages 883-890, XP027770276, ISSN: 0308-8146 [retrieved on 2005-05-01]
- NOOSHKAM MAJID ET AL: "Microwave-assisted isomerisation of lactose to lactulose and Maillard conjugation of lactulose and lactose with whey proteins and peptides", FOOD CHEMISTRY, vol. 200, 31 December 2015 (2015-12-31), pages 1-9, XP029401249, ISSN: 0308-8146, DOI: 10.1016/J.FOODCHEM.2015.12.094
- BRAM VIVIJS ET AL: "Identification of Genes Required for Growth of Escherichia coli MG1655 at Moderately Low pH", FRONTIERS IN MICROBIOLOGY, vol. 7, 25 October 2016 (2016-10-25), XP055385108, DOI: 10.3389/fmicb.2016.01672
- J. P. Kass ET AL: "Browning of Autoclaved Milk Chemical Factors Involved", Industrial and Engineering Chemistry, vol. 32, no. 10, 1 October 1940 (1940-10-01), pages 1360-1366, XP055660544, US ISSN: 0019-7866, DOI: 10.1021/ie50370a019
- Jennewein Biotechnologie Gmbh: "GRAS Notification for T-Fucosyllactose (T-FL)", , 1 March 2015 (2015-03-01), pages 1-24, XP055661223, Retrieved from the Internet: URL:http://wayback.archive-it.org/7993/201 71031041333/https://www.fda.gov/downloads/ Food/IngredientsPackagingLabeling/GRAS/Not iceInventory/UCM461885.pdf [retrieved on 2020-01-23]
- I. Martinez-Castro ET AL: "Influence of thermal processing on carbohydrate composition of milk. Formation of epilactose.", Milchwissenschaft, vol. 35, no. 1, 1 January 1980 (1980-01-01), pages 5-8, XP055720606,
- Geier H ET AL: "Formation of lactulose during heat treatment of milk", Milchwissenschaft, vol. 38, no. 8, 1 January 1983 (1983-01-01), pages 475-477, XP055867726,

## Description

The present invention relates to methods involving a thermal treatment of an aqueous solution containing at least one reducing saccharide. More specifically, the invention relates to methods of preventing isomerization of the reducing saccharide in an aqueous solution upon a thermal treatment of aqueous solution containing the reducing saccharide.

### Background

Maintaining a sterile environment is often a prerequisite for cultivating cells in biotechnological production processes. However, these processes are often prone to foreign growth by adventitious bacteria, fungi or viruses. Being contaminated with adventitious microorganisms has a severe impact on the manufacturing process as it impairs productivity (decreased production capacity due to a degradation or modification of the raw materials, the desired product or the desired biomass, and extended shut down periods of the bioreactor for removal of the contaminant), product quality and product safety (through contaminations of the final product due to the foreign growth itself and/or due to metabolites produced by the undesired microorganisms).

Preventing foreign growth on a biological product as well as in the process of its production is challenging due to the ubiquitous nature of microorganisms and multiple points of microbial entry into the production process. Various methods have been developed for sterilizing equipment (e.g. the interior of a fermenter), growth media as well as any supplements to the growth media that have to be used in a biotechnological production process.

Several methods are known for sterilizing compounds or compositions and are employed to eliminate adventitious microorganisms from supplies to be used in biotechnological production processes. Such methods include gas sterilization (e.g. with ozone or ethylene oxide), radiation sterilization (e.g. ultra violet radiation or gamma radiation), bright light/pulsed light sterilization as well as sterile filtration of liquids and solutions, in particular of liquids and solutions that are heat-sensitive or contain at least one heat-sensitive compound.

Heat treatment of any heat-tolerant supplies is known to be the most reliable and effective sterilization method and wet heat is most widely used for achieving heat sterilization. Wet heat (steam) sterilization means exposing the components to be sterilized to pressurized steam for some time. Typical wet heat sterilization protocols that are used for sterilizing equipment and supplies subject them to highpressure saturated steam at 115 °C to 140 °C for around 60 to 3 minutes. These sterilization protocols may vary depending on the bioburden and nature of the raw material, solution or surface to be sterilized.

Fundamentally, damaging a component by an inappropriate sterilization method may affect quality, safety or productivity of a biotechnological production process, thus, has to be avoided. Especially fluids may undergo various chemical reactions due to sterilization methods using heat, irradiation or chemicals.

The complexity of such chemical reactions is exemplified by heat-treating milk which is typically employed to reduce the microbial load or to inactivate enzymes in the milk, and hence extend the shelf-life of milk. Maillard reactions are known to occur which affect the color and taste of the milk upon its conventional sterilization at 121.1 °C for 20 minutes. Furthermore, the denaturation or inactivation of proteins or vitamins as well as reactions of aldo sugars with amino acids or amino group containing substances is known to occur due to the heat treatment of milk. Besides, it is also well known that lactose (4-*O*-β-D-galactopyranosyl-D-glucopyranose, CAS-number: 63-42-3), a substantial constituent of milk, isomerizes to lactulose (4-*O*-β-D-galactopyranosyl-D-fructofuranose, CAS-number: 4618-18-2) due to heat treatment and gets further degraded to glucose and galactose as well as to further degradation products such as acids. This type of isomerization is favored by basic pH and is also known as the Lobry de Bruyn-Alberda van Ekenstein transformation.

In attempts to limit the undesired effects of heat to the quality and composition of milk, alternative methods of sterilization are used such as "ultra-high-temperature treatment", wherein the milk is exposed to 140 °C for a couple of seconds to few minutes, or "low temperature pasteurization" wherein milk is heated to a temperature of up to 74 °C. Nevertheless, the undesired heat-mediated reactions also occur during these alternative sterilization methods, although to a lesser extent.

This could be further improved by adjusting the pH values of raw skim milk, having an initial pH of 6.70, to values of between 6.59 and 6.72 prior to its sterilization at 120 °C for 10 minutes. This led to a reduced lactulose formation by 28 % at pH 6.59 and a 9 % increased lactulose formation at pH 6.72 as compared to lactulose formation in the original milk.

WO 2015/150328 A1 discloses a method for totally fermentation of oligosaccharides. Further, GRAS notice (GRN) No. 571 (Jennewein Biotechnologie GmbH, March 2015) discloses material and methods for producing 2'-fucosyllactose.

In the biotechnological production of human milk oligosaccharides such as 2'-fucosyllactose, 3-fucosyllactose, lacto-N-tetraose, lacto-N-neotetraose, 3'-sialyllactose or 6'-sialyllactose, lactose is typically used as initial acceptor molecule for further glycosylation steps leading to the desired HMO to be produced. For preventing foreign growth in the fermentation broth, the lactose being supplied has to be sterilized. However, the presence of lactulose within the fermentation broth has to be avoided since it is a known laxative that should not be present in infant formula or any other nutritional product being supplemented with said HMOs. Furthermore, lactulose might be used by the HMO producing bacteria as an alternative acceptor molecule, thus, leading to oligosaccharides which are not present in nature.

As an alternative to heat treatment, it is possible to sterilize a lactose solution by filtration. Typically, solutions containing between 1 mM and 1 M lactose are sterile filtered. However, sterilizing large amounts of a solution by filtration as necessary for industrial scale production is costly, time consuming and less reliable as compared to heat sterilization. Therefore, a reliable, more convenient way of sterilizing lactose with no or only conversion of minute amounts of lactose to lactulose is needed.

The object has been achieved by a method as claimed in the attached claims, wherein an acidic pH of a lactose solution is adjusted prior to and/or in the course of exposing the lactose solution to heat, notwithstanding that the principle of acidifying a sugar solution prior to its heat treatment can be applied to other saccharides than lactose as well.

### Summary

In a first aspect, the present invention provides a method of inhibiting isomerization of a reducing saccharide in an aqueous solution containing said reducing saccharide upon thermal treatment of said aqueous solution by acidifying the aqueous solution prior to and/or in the course of its thermal treatment, as claimed in the attached claims.

In a another aspect, the present invention provides the use of a thermally treated aqueous solution containing at least one reducing saccharide in a biotechnological production of a biological product as claimed in the attached claims.

### Brief description of the drawings

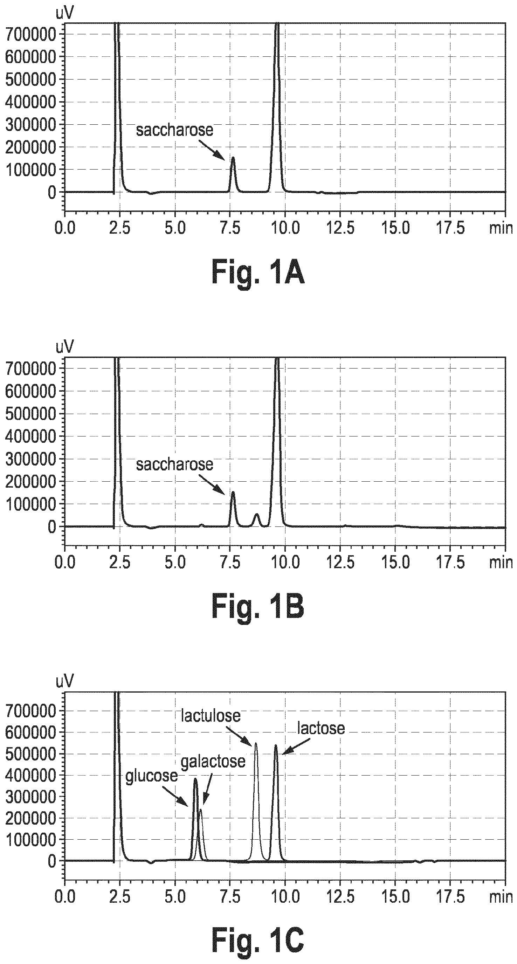

FIG. 1 displays chromatograms of an aqueous solution containing lactose (A) prior to heat sterilization and (B) after heat sterilization. The aqueous solution was not acidified prior to its heat sterilization. FIG. 1C shows a chromatogram of various specific saccharides used as standards.

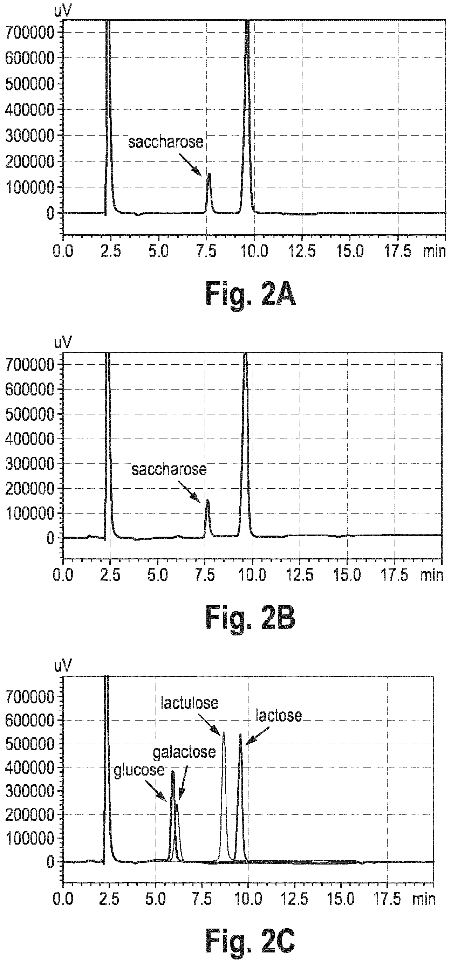

FIG. 2 displays chromatograms of an aqueous solution containing lactose (A) prior to heat sterilization and (B) after heat sterilization. The aqueous solution was acidified prior to its heat sterilization by adding sulfuric acid to the aqueous solution containing lactose. FIG. 2C shows a chromatogram of various specific saccharides used as standards.

### Detailed description

According to the first aspect, provided is a method of inhibiting isomerization of a reducing saccharide in an aqueous solution containing said reducing saccharide (aqueous saccharide solution) upon thermal treatment of said aqueous saccharide solution, the method comprising the step of acidifying the aqueous saccharide solution prior to and/or in the course of its thermal treatment as claimed in the attached claims.

The term "reducing saccharide" as used herein refers to any sugar or saccharide that is capable of acting as a reducing agent because it has a free aldehyde group. The reducing saccharide comprises monosaccharides, disaccharides and oligosaccharides. All monosaccharides are reducing sugars, they can be classified into aldoses, which have an aldehyde group, and the ketoses, which have a ketone group. Ketoses must first tautomerize to aldoses before they can act as reducing sugars. Disaccharides are formed from two monosaccharide residues and oligosaccharides are formed from three to seven monosaccharide residues. Disaccharides and oligosaccharides can be classified as either reducing or nonreducing. Reducing disaccharides like lactose and maltose have only one of their two anomeric carbons involved in the glycosidic bond, meaning that they can convert to an open-chain form with an aldehyde group.

In an additional and/or alternative embodiment, the reducing saccharide is selected from the group consisting of aldoses, disaccharides and oligosaccharides.

The term "aldose" as used herein refers to monosaccharides that contain only one aldehyde group per molecule. Examples of aldoses are D-(+)-glyceraldehyde, D-(-)-erythrose, D-(-)-threose, D-(-)-ribose, D-(-)-arabinose, D-(+)-xylose, D-(-)-Iyxose, D-(+)-allose, D-(+)-altrose, D-(+)-glucose, D-(+)-mannose, D-(-)-gulose, D-(-)-idose, D-(+)-galactose, and D-(+)-talose.

In an additional and/or alternative embodiment, the disaccharide is selected from the group consisting of lactose, maltose, trehalose, cellobiose, chitobiose, kojibiose, nigerose, isomaltose, sophorose, laminaribiose, gentibiose, turanose, matulose, palatinose, gentibiose, mannobiose, melibiose, melibiulose, rutinose, rutinulose and xylobiose.

The term "oligosaccharide" as used herein refers to saccharides consisting of 3, 4, 5, 6, or 7 monosaccharide residues, and thus comprises trisaccharides, tetrasaccharides, pentasaccharides, hexasaccharides and heptasaccharides.

For obtaining the aqueous saccharide solution, an amount of the reducing saccharide is dissolved in a supply of water. In a preferred embodiment, the water is distilled water, double distilled water or deionized water. Dissolution of the reducing saccharide in the water can be facilitated by stirring the water while the reducing saccharide is added or by mixing or shaking the water after the reducing saccharide had been added to the supply of water.

In an embodiment of the method, the aqueous saccharide solution is acidified to a pH having a value of between about 1 to about 6, preferably to a pH having a value of between about 2 to about 5, and more preferably to a pH having a value of between about 3 and about 4. A pH of the aqueous saccharide solution having a value of between about 3 to about 5 was found to be of particular advantage, because isomerization of the reducing saccharide is inhibited or even prevented while formation of degradation products of said reducing saccharide is negligible.

According to the invention, the aqueous saccharide solution is acidified by adding at least one acid to the aqueous saccharide solution. The acid can be any acid which does not lead to an undesired chemical reaction with the reducing saccharide. An example of such an undesired chemical reaction is the formation of mucic acid if nitric acid is added to an aqueous lactose solution.

The acid can be selected from the group of organic acids and inorganic acids, with the provision that the inorganic acid is not nitric acid (or nitrous acid) if the reducing saccharide is galactose or a galactose-containing saccharide, as nitric acid oxidation of galactose or galactose-containing compounds such as lactose leads to mucic acid.

In an additional and/or alternative embodiment, the at least one acid for acidifying the aqueous saccharide solution is an inorganic acid or mineral acid. The inorganic acid is a suitable inorganic acid which - at the amount to be added to the aqueous saccharide solutions - does not inadvertently react with the saccharide. For example, adding nitric acid to an aqueous lactose solution may lead to mucic acid. The inorganic acid is preferably selected from the group consisting of hydrochloric acid, sulfuric acid, sulfurous acid, phosphoric acid, boric acid, hydrofluoric acid, hydrobromic acid, perchloric acid, hydroiodic acid, and carbonic acid.

In the embodiment, wherein the inorganic acid is carbonic acid, the aqueous saccharide solution can be acidified in that the aqueous saccharide solution is gassed with carbon dioxide in a pressurized container.

In an additional and/or alternative embodiment, the at least one acid for acidifying the aqueous saccharide solution is an organic acid. The organic acid may be selected from the group consisting of monocarboxylic acids, dicarboxylic acids, and tricarboxylic acids.

In an additional and/or alternative embodiment, the monocarboxylic acid is selected from, but not limited to, the group consisting of carbonic acid, formic acid (methanoic acid), acetic acid (ethanoic acid), proprionic acid (propanoic acid), butyric acid (butanoic acid), and valeric acid (pentanoic acid).

In an additional and/or alternative embodiment, the dicarboxylic acid is selected from the group consisting of oxalic acid, malonic acid, succinic acid, glutaric acid, adipic acid, maleic acid, malic acid, fumaric acid, glutaconic acid, muconic acid, and citraconic acid.

In an additional and/or alternative embodiment, the tricarboxylic acid is selected from the group consisting of citric acid, isocitric acid, and aconitic acid.

Adjusting the pH of the saccharide solution to an acidic value permits a thermal treatment of the reducing saccharide in the aqueous solution without or at least with a reduced isomerization of the reducing saccharide.

Thermal treatment of the aqueous saccharide solution comprises heating the aqueous saccharide solution after acidification and/or while acidifying as claimed in the attached claims.

Heating and/or keeping an aqueous solution containing a reducing saccharide at such elevated temperatures without isomerization or with significantly reduced isomerization of the reducing saccharide provides multiple advantages such as - for example - the option of dissolving a higher amount of the saccharide in a given amount of water thereby increasing the saccharide concentration in the aqueous solution and reducing the volume of an aqueous saccharide solution to be supplied to a batch for obtaining a desired final saccharide concentration. Additionally and/or alternatively, the viscosity of the aqueous saccharide solution can be decreased by heating/keeping the aqueous saccharide solution at an elevated temperature, thereby facilitating handling and/or pumping of the aqueous saccharide solution, e.g. through a membrane filter.

According to the invention, "thermal treatment" comprises heating the aqueous saccharide solution to a temperature in the range of 115 °C to 150 °C and keeping the temperature for up to about 60 minutes, autoclaving the aqueous saccharide solution at 121.1 °C for 15 to 30 minutes; heating the aqueous solution to a temperature of 130 °C to 150 °C and holding the temperature for up to 5 minutes; or subjecting the aqueous solution to a temperature of between 71.5 °C to 74 °C, for 15 to 30 seconds.

Autoclaving is one of the most important methods of germ destruction wherein saturated, superheated steam is utilized. The condensation of steam on the object to be sterilized releases energy which causes irreversible damage to the microorganisms. To this end, the interior of the autoclave is vented during the intial rise time. In doing so, the atmospheric air is displaced from the interior and replaced by saturated, superheated steam. Venting takes place using a flow process or through fractioned venting; once venting is complete, the vent valve is closed. This marks the start of the compensation time. After this period, every point of the item to be sterilised reaches the required temperature due to the effect of the saturated steam. After this, the actual sterilisation phase begins. The duration of sterilisation is dependent on both germ loading and sterilisation temperature. Autoclaving at 121.1 °C (250 °F) for 15 minutes to 30 minutes is seen as standard.

In an alternative thermal treatment for sterilizing the aqueous saccharide solution according to the invention, the aqueous saccharide solution is sterilized by a process called "ultra-high-temperature treatment", a continuous sterilization method, which comprises heating the aqueous to a temperature of 130 °C - 150 °C with 140 °C as a main point. The corresponding holding time may vary from 8 to 40 seconds, occasionally to up to 5 minutes, depending on the properties of the solution to be sterilized.

In an alternative thermal treatment for sterilizing the aqueous saccharide solution according to the invention, the aqueous saccharide solution is subjected to a high temperature/short time (HTST) pasteurization, in which the solution is heated to a temperature of between 71.5 °C to 74 °C, preferably to 72 °C for about 15 seconds to about 30 seconds, and is moved in a controlled, continuous flow while subjected to said thermal treatment.

In yet another embodiment of the thermal treatment for sterilizing the aqueous saccharide solution, the aqueous saccharide solution is subjected to "flash pasteurization", wherein the aqueous saccharide solution is subjected to 71.7 °C for 15 seconds.

Acidifying an aqueous solution of a reducing saccharide prior to subjecting the aqueous saccharide solution to any of these thermal treatments and/or in the course of its thermal treatment for sterilizing the aqueous saccharide solution inhibits or even prevents isomerization of the reducing saccharide in the aqueous solution upon its heat treatment.

With the method of inhibiting isomerization as claimed in the attached claims, a thermally treated aqueous solution containing at least one reducing saccharide can be provided.

The thermally treated aqueous solution contains at least one reducing saccharide and is obtained by the acidification of the aqueous saccharide solution prior to and/or in the course of its thermal treatment as claimed in the attached claims and contains no or at least less amounts of undesired isomerization products of said at least one reducing saccharide as compared to a similar aqueous solution of the same reducing saccharide which was not acidified prior to an identical thermal treatment.

According to the invention, the aqueous solution containing a reducing saccharide is a sterile aqueous solution. The sterile aqueous solution containing a reducing saccharide is obtained by the method of inhibiting isomerization of said reducing saccharide as claimed in the attached claims, including the thermal treatment of said aqueous saccharide solution for sterilizing said aqueous saccharide solution. Thus, aqueous solution has been sterilized by the thermal treatment.

The aqueous solution obtained by the method according to the attached claims and containing a reducing saccharide can have an elevated temperature, i.e. a temperature of about 30 °C, about 40 °C, about 50 °C, about 60 °C, about 70 °C or even about 80 °C, and contains the reducing saccharide in an amount that is higher than the amount of said reducing saccharide that can be dissolved in water at room temperature. With the method according to the attached claims, an aqueous solution containing lactose in a concentration of ≥ 10 mM, preferably in a concentration of ≥ 100 mM, more preferably in a concentration of ≥ 0.66 M, most preferably in a concentration of of ≥ 1 M, can be provided if the temperature of the acidified aqueous lactose solution is maintained at about 40 °C to about 60 °C.

The aqueous saccharide solution containing at least one reducing saccharide, such as - for example - lactose, in an amount that is higher than the amount of the saccharide that can be dissolved in water at room temperature may be a sterile aqueous lactose solution that has been obtained by the method as claimed in the attached claims and allowed to cool down to the desired elevated temperature at which the sterile aqueous saccharide solution is kept.

The invention also concerns the use of a sterile aqueous solution containing at least one reducing saccharide obtained by a method as claimed in the attached claims in a biotechnological production of a human milk oligosaccharide.

According to the invention, the use of the thermally treated aqueous solution containing a reducing saccharide obtained by a method as claimed in the attached claims comprises the use in a fermentative production process. The term "fermentative production process" as used herein refers to a process wherein microorganisms are grown in a medium or broth with the aim of producing a biological product or specialty product that is synthesized by the microorganisms.

Using a thermally treated aqueous solution containing at least one reducing saccharide, wherein the aqueous saccharide solution has been acidified as described herein before prior to and/or in the course of the thermal treatment of the aqueous saccharide solution as claimed in the attached claims is advantageous, among others, in that no or less undesired isomerization products of the reducing saccharide are present in the aqueous saccharide solution, and that no or less undesired isomerization products are supplied to the biotechnological production process as compared to a similar aqueous saccharide solution that was not acidified prior to its thermal treatment. Thus, there is no necessity remove the undesired isomerization products from the heat treated aqueous saccharide solution before it can be used in a biotechnological production process.

"Fermentation" or "fermentative" refers to the bulk growth of microorganisms on or in a growth medium (fermentation broth) with the goal of producing a specific chemical product, the "biological product", presently: a human milk oligosaccharide. To this end, cells of one or a limited number of strains of microorganisms are grown in a bioreactor (fermenter) under optimum conditions for the microorganisms to perform the desired production with limited production of undesired impurities. The environmental conditions inside the bioreactor, such as temperature, nutrient concentrations, pH, and dissolved gases (especially oxygen for aerobic fermentations) affect the growth and productivity of the organisms, and are therefore monitored, controlled and adjusted if necessary.

Thus, according to the invention the method of fermentative production of a human milk oligosaccharide, in which the sterile aqueous saccharide solution obtained by the method of the invention is used, comprises the steps of:
- providing a cell that is capable of producing the human milk oligosaccharide;
- cultivating the at least one cell in a fermentation broth containing and/or being supplemented with the sterile aqueous solution containing the at least one reducing saccharide, wherein the reducing saccharide is present in the sterile solution in a concentration of ≥ 0.66 M, for the at least one cell to produce the biological product; and
- optionally purifying the biological product from the fermentation broth,
wherein the human milk oligosaccharide is produced in an amount of ≥ 100 g/L in the fermentation broth, in an amount of ≥ 150 g/L in the fermentation broth, and in an amount of ≥ 200 g/L in the fermentation broth at the end of the fermentation process.

In an embodiment of the use according to the invention, said living cell is a prokaryotic cell or a eukaryotic dell. Appropriate cells include yeast, bacteria, archaebacteria, fungi, insect cells, plant cells and animal cells, including mammalian cells (such as human cells and cell lines).

In an additional and/or alternative embodiment, the prokaryotic cell is a bacterial cell, preferably selected from the genus selected from the group consisting of *Bacillus, Lactobacillus, Lactococcus, Enterococcus, Bifidobacterium, Sporolactobacillus spp., Micromomospora spp., Micrococcus spp., Rhodococcus spp.,* and *Pseudomonas.* Suitable bacterial species are *Bacillus subtilis, Bacillus licheniformis, Bacillus coagulans, Bacillus thermophilus, Bacillus laterosporus, Bacillus megaterium, Bacillus mycoides, Bacillus pumilus, Bacillus lentus, Bacillus cereus, Bacillus circulans, Bifidobacterium longum, Bifidobacterium infantis, Bifidobacterium bifidum, Citrobacter freundii, Clostridium cellulolyticum, Clostridium Ijungdahlii, Clostridium autoethanogenum, Clostridium acetobutylicum, Corynebacterium glutamicum, Enterococcus faecium, Enterococcus thermophiles, Escherichia coli, Erwinia herbicola (Pantoea agglomerans), Lactobacillus acidophilus, Lactobacillus salivarius, Lactobacillus plantarum, Lactobacillus helveticus, Lactobacillus delbrueckii, Lactobacillus rhamnosus, Lactobacillus bulgaricus, Lactobacillus crispatus, Lactobacillus gasseri, Lactobacillus casei, Lactobacillus reuteri, Lactobacillus jensenii, Lactococcus lactis, Pantoea citrea, Pectobacterium carotovorum, Proprionibacterium freudenreichii, Pseudomonas fluorescens, Pseudomonas aeruginosa, Streptococcus thermophiles* and *Xanthomonas campestris.*

In an additional and/or alternative embodiment, the eukaryotic cell is a yeast cell, an insect cell, a plant cell or a mammalian cell. The yeast cell is preferably selected from the group consisting of *Saccharomyces sp.,* in particular *Saccharomyces cerevisiae, Saccharomycopsis sp., Pichia sp.,* in particular *Pichia pastoris, Hansenula sp., Kluyveromyces sp., Yarrowia sp., Rhodotorula sp.,* and *Schizosaccharomyces sp.*

In an embodiment of the use according to the invention, said human milk oligosaccharide may be selected from the group consisting of 2'-fucosyllactose, 3-fucosyllactose, 2',3-difucosyllactose, lacto-*N*-triose II, lacto-*N*-tetraose, lacto-*N-*neotetraose, lacto-*N*-fucopentaose I, lacto-*N*-neofucopentaose I, lacto-*N-*fucopentaose II, lacto-*N*-fucopentaose III, lacto-*N*-fucopentaose V, lacto-*N-*neofucopentaose V, lacto-*N*-difucohexaose I, lacto-*N*-difucosylhexaose II, *para-*Lacto-*N*-fucosylhexaose, fucosyl-lacto-*N*-sialylpentaose b, fucosyl-lacto-*N-*sialylpentaose c, fucosyl-lacto-*N*-sialylpentaose c, disialyl-lacto-*N*-fucopentaose, 3-fucosyl-3'-sialyllactose, 3-fucosyl-6'-sialyllactose, lacto-*N*-neodifucohexaose I, 3'-sialyllactose, 6'-sialyllactose, sialyllacto-*N*-tetraoses LST-a, LST-b, LST-c, and disialyllacto-*N*-tetraose.

In an additional and/or alternative embodiment of the use and/or the method according to the invention, said reducing saccharide is lactose. This embodiment is of particular advantage wherein lactose has to be supplied to the fermentation broth for the living cells for producing the desired human milk oligosaccharide. Being able to reduce or avoid the formation of lactulose upon heat sterilization of an aqueous lactose solution by the method according to the first aspect, eliminates the need of removing lactulose from the HMO preparation when the HMO preparation shall be used for manufacturing a nutritional formula, especially an infant formula, a medicinal food or a dietary supplement.

Using a thermally treated aqueous saccharide solution containing at least one reducing saccharide, wherein the aqueous saccharide solution has been acidified as described herein before prior to and/or in the course of its thermal treatment according to the attached claims in a fermentative production process provides additional advantages. First, the present invention permits sterilization of an aqueous solution containing a reducing saccharide by heat sterilization methods without or with reduced isomerization of the reducing saccharide. Hence, aqueous solutions containing a reducing saccharide do not have to be sterilized by sterile filtration, which is less reliable than heat sterilization (e.g. with regard to elimination of bacteriophages), in particular if large volumes of an aqueous saccharide solution, such as several cubic meters, need to be sterilized. In addition, heat sterilization of large volumes is more economic than sterile filtration. Second, the present invention permits providing aqueous saccharide solutions containing at least one reducing saccharide, wherein the concentrations of the at least one reducing saccharide is higher than the saturation concentration of the at least one reducing saccharide at room temperature, as the aqueous saccharide solution can be heated and kept at an elevated temperature, i.e. a temperature above room temperature. Moreover, keeping an aqueous saccharide solution at an elevated temperature reduces the viscosity of the aqueous saccharide solution which in turn eases handling of the aqueous saccharide solution, for example when pumping the aqueous saccharide solution through a pipe or a hose. Third, being able to provide an aqueous saccharide solution having an increased saccharide concentration permits obtaining higher product yields in a fermentative production process. This is because the volume of a fermenter is limited and the volume of the fermentation broth in a fermenter increases during a fermentation process due to the supply of - among others - an aqueous saccharide solution to the fermentation broth which aqueous saccharide solution is required for the production of the desired biological product by the cells being cultivated. The higher the concentration of the saccharide in the aqueous saccharide solution the smaller the volume of the aqueous saccharide solution that needs to be added to a fermentation broth in order to obtain and/or maintain a predetermined concentration of the at least one reducing saccharide in the fermentation broth. Thus, using an aqueous saccharide solution according to the invention having an increased saccharide concentration, a higher saccharide concentration in the fermentation broth in a given fermenter can be achieved or a predetermined saccharide concentration can be maintained for a longer time period as the volume in the fermenter being available for supplies is depleted more slowly. This in turn provides more of the reducing saccharide to the cells for producing the desired biological product, and hence increases the yield of the desired biological product that can be obtained in a single batch fermentation. Therefore, the desired human milk oligosaccharide can be produced - at the end of the fermentation process - in an amount of ≥ 100 g/L in the fermentation broth, preferably in an amount of ≥ 150 g/L in the fermentation broth, more preferably in an amount of ≥ 200 g/L in the fermentation broth. For example amounts of 2'-fucosyllactose of more than 100 g/L, namely of about 150 g/L were obtained when a acidified 0.66 M aqueous lactose solution was used which was heat sterilized, and even higher yields can be obtained if the lactose concentration in the aqueous solution to be fed to the fermentation broth is further increased.

In an embodiment, the human milk oligosaccharide is selected from the group consisting of
2'-fucosyllactose, 3-fucosyllactose, 2',3-difucosyllactose, lacto-*N*-triose II, lacto-*N-*tetraose, lacto-*N*-neotetraose, lacto-*N*-fucopentaose I, lacto-*N*-neofucopentaose I, lacto-*N*-fucopentaose II, lacto-*N*-fucopentaose III, lacto-*N*-fucopentaose V, lacto-*N-*neofucopentaose V, lacto-*N*-difucohexaose I, lacto-*N*-difucosylhexaose II, *para-*Lacto-*N*-fucosylhexaose, fucosyl-lacto-*N*-sialylpentaose b, fucosyl-lacto-*N-*sialylpentaose c, fucosyl-lacto-*N*-sialylpentaose c, disialyl-lacto-*N*-fucopentaose, 3-fucosyl-3'-sialyllactose, 3-fucosyl-6'-sialyllactose, lacto-*N*-neodifucohexaose I, 3'-sialyllactose, 6'-sialyllactose, sialyllacto-*N*-tetraoses LST-a, LST-b, LST-c, and disialyllacto-*N*-tetraose.

In an alternative embodiment, the human milk oligosaccharide is selected from the group consisting of lactosucrose and lactobionic acid or derivatives of the above mentioned human milk oligosaccharides.

The human milk oligosaccharide produced in the use of the invention, is preferably selected from the group consisting of 2'-fucosyllactose, 3-fucosyllactose, 2',3-difucosyllactose, lacto-*N*-triose II, lacto-*N*-tetraose, lacto-*N*-neotetraose, lacto-*N-*fucopentaose I, lacto-*N*-neofucopentaose I, lacto-*N*-fucopentaose II, lacto-*N-*fucopentaose III, lacto-*N*-fucopentaose V, lacto-*N*-neofucopentaose V, lacto-*N-*difucohexaose I, lacto-*N*-difucosylhexaose II, para-Lacto-*N*-fucosylhexaose, fucosyl-lacto-*N*-sialylpentaose b, fucosyl-lacto-*N*-sialylpentaose c, fucosyl-lacto-*N-*sialylpentaose c, disialyl-lacto-*N*-fucopentaose, 3-fucosyl-3'-sialyllactose, 3-fucosyl-6'-sialyllactose, lacto-*N*-neodifucohexaose I, 3'-sialyllactose, 6'-sialyllactose, sialyllacto-*N*-tetraoses LST-a, LST-b, LST-c, and disialyllacto-*N*-tetraose, and can be used in nutritional formulations, such as infant formula, medicinal food and dietary supplements.

Provided that the reducing saccharide is lactose, the method of inhibiting isomerization according to the invention may provide a heat sterilized lactose solution without or with reduced amounts of lactulose for fermentative production of a human milk oligosaccharide. Said human milk oligosaccharide may then be employed in the manufacturing of a nutritional formulation, preferably an infant formula, which does not contain or contains less amount of epilactose, lactulose and/or a derivative of lactulose such as fucosyllactulose (without the need of removing lactulose or its derivative from the HMO preparation).

The present invention will be described with respect to particular embodiments and with reference to drawings, but the invention is not limited thereto but only by the claims. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

In the description and drawings provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention, which is limited by the attached claims.

### Example 1 - Acidification of lactose with organic acids prior to heat sterilization

A 0.66 M lactose solution was prepared by dissolving 226 g of lactose in water. The final volume of the solution was 1 litre. At a temperature of 30°C to 35°C the pH was adjusted by using 50 % (w/v) citrate or 99 % (v/v) acetic acid. Afterwards, the solution was sterilized in a vertical autoclave (Systec VX-65, Linden, Germany) at 121 °C for 20 minutes. Samples were taken before and after heat sterilization and kept frozen prior to analysis by high performance liquid chromatography (HPLC). HPLC was carried out using a RID-10A refractive index detector (Shimadzu, Germany) and a Waters XBridge Amide Column 3.5 µm (250 x 4.6 mm) (Eschborn, Germany) connected to a Shimadzu HPLC system. Isocratic elution was carried out with 30% solvent A (50% (v/v) acetonitrile in double distilled water, 0.1% (v/v) NH4OH) and 70% solvent B (80% (v/v) acetonitrile in double distilled water, 0.1% (v/v) NH4OH) at 35°C and at a flow rate of 1.4 mL min-1. Samples were cleared by solid phase extraction on an ion exchange matrix (Strata ABW, Phenomenex). Ten microliter of the sample (dilution of 1:5) was applied to the column. Finally, the relative amount of detected sugars was determined. As depicted in tables 1 and 2, the heat sterilization induced lactose isomerization decreased with decreasing pH values of the solutions prior to heat treatment. No lactulose formation could be observed at pH 4.0 to 3.0 or pH 3.0 when acidification was carried out with citrate or acetic acid, respectively. The acid-catalysed degradation of lactose into its monosaccharides was increased with lower pH values but was not detectable at pH 4.5.

**Table 1: Relative amount of sugars detected in pH adjusted 0.66 M lactose solutions before and after heat sterilization. The pH adjustment was carried out using 50 % (w/v) citrate. Depicted is the percental amount of sugars (area under the curve; AUC) detected by HPLC.**

| pH | Relative composition [%] | | |
|---|---|---|---|
| | Monosaccharides | Lactulose | Lactose |
| **before heat sterilization** | | | |
| 3.0 - 6.8 | n. d. | n. d. | 100 |

| **after heat sterilization** | | | |
|---|---|---|---|
| 6.8 (no pH adjustment) | 0.44 | 6.16 | 93.40 |
| 5.0 | 0.27 | 0.87 | 98.85 |
| 4.5 | n. d. | 0.15 | 99.85 |
| 4.0 | 0.68 | n. d. | 99.32 |
| 3.5 | 1.87 | n. d. | 98.13 |
| 3.0 | 5.96 | n. d. | 94.04 |

**Table 2: Relative amount of sugars detected in pH adjusted 0.66 M lactose solutions before and after heat sterilization. The pH adjustment was carried out using 80 % (v/v) acetic acid. Depicted is the percental amount of sugars (area under the curve; AUC) detected by HPLC.**

| pH | Relative composition [%] | | |
|---|---|---|---|
| | Monosaccharides | Lactulose | Lactose |
| **before heat sterilization** | | | |
| 3.0 - 6.8 | n. d. | n. d. | 100 |

| **after heat sterilization** | | | |
|---|---|---|---|
| 6.8 (no pH adjustment) | 0.44 | 6.16 | 93.40 |
| 5.0 | 0.21 | 1.13 | 98.65 |
| 4.5 | n. d. | 0.31 | 99.69 |
| 4.0 | 0.49 | 0.12 | 99.39 |
| 3.5 | 1.41 | 0.07 | 98.52 |
| 3.0 | 4.35 | n. d. | 95.65 |

### Example 2 - Acidification of lactose with inorganic acids prior to heat sterilization

A 0.66 M lactose solution was prepared by dissolving 226 g of lactose in water. The final volume of the solution was 1 litre. At a temperature of 30 °C to 35 °C the pH was adjusted by using 37 % (v/v) hydrochloric acid, 50 % (v/v) phosphoric acid or 96 % (v/v) sulfuric acid. Afterwards, the solution was sterilized in a vertical autoclave (Systec VX-65, Linden, Germany) at 121 °C for 20 minutes. Samples were taken before and after heat sterilization and kept frozen prior to analysis by high performance liquid chromatography (HPLC). HPLC was carried out using a RID-10A refractive index detector (Shimadzu, Germany) and a Waters XBridge Amide Column 3.5 µm (250 x 4.6 mm) (Eschborn, Germany) connected to a Shimadzu HPLC system. Isocratic elution was carried out with 30% solvent A (50% (v/v) acetonitrile in double distilled water, 0.1% (v/v) NH4OH) and 70% solvent B (80% (v/v) acetonitrile in double distilled water, 0.1% (v/v) NH4OH) at 35°C and at a flow rate of 1.4 mL min-1. Samples were cleared by solid phase extraction on an ion exchange matrix (Strata ABW, Phenomenex). Ten microliter of the sample (dilution of 1:5) was applied to the column. Finally, the relative amount of detected sugars was determined. As depicted in tables 3, 4 and 5, the heat sterilization induced lactose isomerization decreased in consequence of increased acidification of the solutions prior to heat treatment. No lactulose formation could be observed at pH 3.5 to 3.0. Contrarily, the acid-catalysed degradation of lactose into its monosaccharides was increased with lower pH values but was absent at pH values 4.5 to 4.0 or pH 5.0 to 4.0 when phosphoric acid and sulfuric acid or hydrochloric acid was used for acidification, respectively.

**Table 3: Relative amount of sugars detected in pH adjusted 0.66 M lactose solutions before and after heat sterilization. The pH adjustment was carried out using 37 % (v/v) hydrochloric acid. Depicted is the percental amount of sugars (area under the curve; AUC) detected by HPLC.**

| pH | Relative composition [%] | | |
|---|---|---|---|
| | Monosaccharides | Lactulose | Lactose |
| **before heat sterilization** | | | |
| 3.0 - 6.8 | n. d. | n. d. | 100 |

| **after heat sterilization** | | | |
|---|---|---|---|
| 6.8 (no pH adjustment) | 0.44 | 6.16 | 93.40 |
| 5.0 | n. d. | 0.57 | 99.43 |
| 4.5 | n. d. | 0.17 | 99.83 |
| 4.0 | n. d. | 0.20 | 99.80 |
| 3.5 | 1.50 | n. d. | 98.50 |
| 3.0 | 5.62 | n. d. | 94.38 |

**Table 4: Relative amount of sugars detected in pH adjusted 0.66 M lactose solutions before and after heat sterilization. The pH adjustment was carried out using 50 % (v/v) phosphoric acid. Depicted is the percental amount of sugars (area under the curve; AUC) detected by HPLC.**

| pH | Relative composition [%] | | |
|---|---|---|---|
| | Monosaccharides | Lactulose | Lactose |
| **before heat sterilization** | | | |
| 3.0 - 6.8 | n. d. | n. d. | 100 |

| **after heat sterilization** | | | |
|---|---|---|---|
| 6.8 (no pH adjustment) | 0.44 | 6.16 | 93.40 |
| 5.0 | 0.30 | 0.49 | 99.21 |
| 4.5 | n. d. | 0.10 | 99.90 |
| 4.0 | n. d. | 0.08 | 99.92 |
| 3.5 | 1.40 | n. d. | 98.60 |
| 3.0 | 4.44 | n. d. | 95.56 |

**Table 5: Relative amount of sugars detected in pH adjusted 0.66 M lactose solutions before and after heat sterilization. The pH adjustment was carried out using 96 % (v/v) sulfuric acid. Depicted is the percental amount of sugars (area under the curve; AUC) detected by HPLC.**

| pH | Relative composition [%] | | |
|---|---|---|---|
| | Monosaccharides | Lactulose | Lactose |
| **before heat sterilization** | | | |
| 3.0 - 6.8 | n. d. | n. d. | 100 |

| **after heat sterilization** | | | |
|---|---|---|---|
| 6.8 (no pH adjustment) | 0.43 | 6.34 | 93.24 |
| 5.0 | 0.26 | 0.67 | 99.05 |
| 4.5 | n. d. | 0.17 | 99.83 |
| 4.0 | n. d. | 0.17 | 99.82 |
| 3.5 | 1.11 | n. d. | 98.89 |
| 3.0 | 3.21 | n. d. | 96.79 |

### Example 3 - Improved production process of 2'-fucosyllactose

An engineered *E. coli* BL21 (DE3) ΔnagAb ΔwcaJ ΔfucIK ΔpfkA strain was used in accordance with European patent application 16 196 486, overexpressing enzymes for *de novo* synthesis of GDP-Fucose (ManB, ManC, Gmd, WcaG), the bifunctional L-fucokinase/L-fucose 1-phosphat guanylyltranferase of *Bacteroides fragilis,* the 2-fucosyltransferase gene *wbgL* from E. *coli*:O126, the lactose permease gene *lacY,* the sugar efflux transporter yberc0001_*9420* from *Yersinia bercovieri* ATCC 43970, the fructose-1,6-bisphosphate aldolase *(fbaB)* and a heterologous fructose-1,6-bisphosphate phosphatase *(fbpase)* from *Pisum sativum.*

The *E. coli* strain was cultivated in a 3L fermenter at 33°C in a mineral salts medium that contains 3 g/L KH₂PO₄, 12 g/L K₂HPO₄, 5 g/L (NH₄)₂SO₄, 0.3 g/L citric acid, 2 g/L MgSO₄ x 7H₂O, 0.1 g/L NaCl and 0.015 g/L CaCl₂ x 6H₂O with 1 mL/L trace element solution (54.4 g/L ammonium ferric citrate, 9.8 g/L MnCl₂ x 4H₂O, 1.6 g/L CoCl₂ x 6H₂O, 1 g/L CuCl₂ x 2H₂O, 1.9 g/L H₃BO₃, 9 g/L ZnSO₄ x 7H₂O, 1.1 g/L Na₂MoO₄ x 2H₂O, 1.5 g/L Na₂SeO₃, 1.5 g/L NiSO₄ x 6H₂O), 2 % (v/v) glycerol as sole carbon- and energy source as well as 60 mM of heat sterilized lactose, which was acidified to pH 3.0 with 96 % (v/v) sulfuric acid prior to sterilization. The pH was hold at 7.0 by titrating 25% ammonia. The fermenter was inoculated to an OD₆₀₀ of 0.1 with a pre-culture grown in the described medium but lacking lactose. After leaving the batch phase, indicated by a rise in the dissolved oxygen level, the glycerol feed (60% v/v) as well as the 0.66 M lactose feed (acidified to pH 3.0 using 96 %(v/v) sulfuric acid prior to heat sterilization) was started. A concentration of 10-40 mM lactose was held throughout the production phase of the fermentation process, regulated according to HPLC-analyses. Glycerol (60% v/v) was fed with flow rates of 6-8 ml/L/h (referring to the starting volume). The fermentation was stopped when the filling volume in the tank reached its maximum. At this point, a 2'-fucosyllactose titer of 146 g/L was determined in the culture supernatant of the broth.

In course of 2'-fucosyllacose fermentation processes using sterile-filtered lactose, instead of acidified, heat sterilized lactose, comparable 2'-fucosyllactose titers were achieved. Furthermore, the kind and amounts of by-products detected in the culture broth of fermentations carried out with sterile-filtered or heat-sterilized (acidified) lactose were comparable. None of the by-products of the kind lactulose, epilactose, fucosyllactulose or fucosylepilactose as well as no other by-products which may originate from the addition of heat-sterilized lactose, were detected in the broth when acidified, heat-sterilized lactose was provided for the fermentation.

## Claims

1. A method of inhibiting isomerization of a reducing saccharide in an aqueous solution containing said reducing saccharide upon a thermal treatment for sterilizing said aqueous saccharide solution, the method comprising the step of acidifying the aqueous saccharide solution prior to and/or in the course of the thermal treatment, wherein the thermal treatment comprises heating the aqueous saccharide solution to a temperature in the range of 115 °C to 150 °C and keeping the temperature for up to 60 minutes; autoclaving the aqueous saccharide solution at 121.1 °C for 15 to 30 minutes; heating the aqueous solution to a temperature of 130 °C to 150 °C and holding the temperature for up to 5 minutes; or subjecting the aqueous solution to a temperature of between 71.5 °C to 74 °C, for 15 to 30 seconds, and wherein the aqueous saccharide solution is acidified by adding at least one acid to said aqueous saccharide solution.

2. The method according to claim 1, wherein the aqueous saccharide solution is acidified to a pH having a value of between 1 to 6.

3. The method according to claim 1 or 2, wherein the acid is an inorganic acid selected from the group consisting of hydrochloric acid, sulfuric acid, sulfurous acid, phosphoric acid, boric acid, hydrofluoric acid, hydrobromic acid, perchloric acid, hydroiodic acid and carbonic acid.

4. The method according to claim 1 or 2, wherein the acid is an organic acid selected from the group consisting of monocarboxylic acids, dicarboxylic acids, and tricarboxylic acids.

5. The method according to any one of claims 1 to 4, wherein the reducing sugar is selected from the group consisting of aldoses, disaccharides and oligosaccharides, wherein the disaccharide is selected from the group consisting of lactose and maltose.

6. Use of a sterile aqueous saccharide solution containing at least one reducing saccharide obtained by a method as claimed in any of claims 1 to 5, in a method of biotechnological production of a human milk oligosaccharide, the method comprising the steps of:
- providing at least one cell that is capable of producing the human milk oligosaccharide;
- cultivating the at least one cell in a fermentation broth containing and/or being supplemented with the sterile aqueous saccharide solution, wherein the reducing saccharide is present in the sterile solution in a concentration of ≥ 0.66 M, for the at least one cell to produce the human milk oligosaccharide; and
- optionally purifying the biological product from the fermentation broth,
wherein the human milk oligosaccharide is produced in an amount of ≥ 100 g/L in the fermentation broth, in an amount of ≥ 150 g/L in the fermentation broth, and in an amount of ≥ 200 g/L in the fermentation broth at the end of the fermentation process.

7. The use according to claim 6, wherein the reducing saccharide is present in the sterile aqueous saccharide solution in a concentration of ≥ 1 M.

8. The use according to claim 6 or 7, wherein the sterile aqueous solution is a wet heat sterilized aqueous solution containing lactose.

9. The use according to any one of claims 6 to 8, wherein the human milk oligosaccharide is selected from the group consisting of 2'-fucosyllactose, 3-fucosyllactose, 2',3-difucosyllactose, lacto-*N*-triose II, lacto-*N*-tetraose, lacto-*N-*neotetraose, lacto-*N*-fucopentaose I, lacto-*N*-neofucopentaose I, lacto-*N-*fucopentaose II, lacto-*N*-fucopentaose III, lacto-*N*-fucopentaose V, lacto-*N-*neofucopentaose V, lacto-*N*-difucohexaose I, lacto-*N*-difucosylhexaose II, *para-*Lacto-*N*-fucosylhexaose, fucosyl-lacto-*N*-sialylpentaose b, fucosyl-lacto-*N-*sialylpentaose c, fucosyl-lacto-*N*-sialylpentaose c, disialyl-lacto-*N*-fucopentaose, 3-fucosyl-3'-sialyllactose, 3-fucosyl-6'-sialyllactose, lacto-*N*-neodifucohexaose I, 3'-sialyllactose, 6'-sialyllactose, sialyllacto-*N*-tetraoses LST-a, LST-b, LST-c, and disialyllacto-*N*-tetraose.

## Patentansprüche

1. Verfahren zur Hemmung der Isomerisierung eines reduzierenden Saccharids in einer wässrigen Lösung, die das reduzierende Saccharid enthält, bei einer thermischen Behandlung zur Sterilisierung der wässrigen Saccharidlösung, wobei das Verfahren den Schritt des Ansäuerns der wässrigen Saccharidlösung vor und / oder während der thermischen Behandlung umfasst, wobei die thermische Behandlung Erhitzen der wässrigen Saccharidlösung auf eine Temperatur im Bereich von 115 °C bis 150 °C und das Halten der Temperatur für bis zu 60 Minuten umfasst; Autoklavieren der wässrigen Saccharidlösung bei 121. 1 °C für 15 bis 30 Minuten umfasst; Erhitzen der wässrigen Lösung auf eine Temperatur von 130 °C bis 150 °C und Halten der Temperatur für bis zu 5 Minuten umfasst; oder Aussetzen der wässrigen Lösung einer Temperatur zwischen 71.5 °C und 74 °C für 15 bis 30 Sekunden umfasst,
und wobei die wässrige Saccharidlösung durch Zugabe mindestens einer Säure zu der wässrigen Saccharidlösung angesäuert wird.

2. Verfahren nach Anspruch 1, wobei die wässrige Saccharidlösung auf einen pH-Wert zwischen 1 und 6 angesäuert wird.

3. Verfahren nach Anspruch 1 oder 2, wobei die Säure eine anorganische Säure ist, ausgewählt aus der Gruppe bestehend aus Salzsäure, Schwefelsäure, schwefliger Säure, Phosphorsäure, Borsäure, Fluorwasserstoffsäure, Bromwasserstoffsäure, Perchlorsäure, lodwasserstoffsäure und Kohlensäure.

4. Verfahren nach Anspruch 1 oder 2, wobei die Säure eine organische Säure ist, ausgewählt aus der Gruppe bestehend aus Monocarbonsäuren, Dicarbonsäuren und Tricarbonsäuren.

5. Verfahren nach einem der Ansprüche 1 bis 4, wobei das reduzierende Saccharid ausgewählt ist aus der Gruppe bestehend aus Aldosen, Disacchariden und Oligosacchariden, wobei das Disaccharid ausgewählt ist aus der Gruppe bestehend aus Lactose und Maltose.

6. Verwendung einer sterilen wässrigen Saccharidlösung, die mindestens ein reduzierendes Saccharid enthält, die durch ein Verfahren nach einem der Ansprüche 1 bis 5 erhalten wurde, in einem Verfahren zur biotechnologischen Herstellung eines humanen Milch-Oligosaccharids, wobei das Verfahren die folgenden Schritte umfasst:
- Bereitstellen mindestens einer Zelle, die in der Lage ist, das humane Milch-Oligosaccharid herzustellen;
- Kultivieren der mindestens einen Zelle in einer Fermentationsbrühe, die die sterile wässrige Saccharidlösung enthält und/oder mit dieser versehen wird, wobei das reduzierende Saccharid in der sterilen Lösung in einer Konzentration von ≥ 0,66 M vorhanden ist, damit die mindestens eine Zelle das humane Milch-Oligosaccharid produziert; und
- optionales Aufreinigen des humanen Milch-Oligosaccharids aus der Fermentationsbrühe,
wobei das humane Milch-Oligosaccharid am Ende des Fermentationsverfahrens in einer Menge von ≥ 100 g/L in der Fermentationsbrühe, in einer Menge von ≥ 150 g/L in der Fermentationsbrühe, und in einer Menge von ≥ 200 g/L in der Fermentationsbrühe hergestellt wird.

7. Verwendung nach Anspruch 6, wobei das reduzierende Saccharid in der sterilen wässrigen Saccharidlösung in einer Konzentration von ≥ 1 M vorhanden ist.

8. Verwendung nach Anspruch 6 oder 7, wobei die sterile wässrige Lösung eine mit feuchter Hitze sterilisierte wässrige Lösung enthaltend Lactose ist.

9. Verwendung nach einem der Ansprüche 6 bis 8, wobei das humane Milch-Oligosaccharid ausgewählt ist aus der Gruppe bestehend aus 2'-Fucosyllactose, 3-Fucosyllactose, 2',3-Difucosyllactose, Lacto-*N*-triose II, Lacto-*N*-tetraose, Lacto-*N*-neotetraose, Lacto-*N*-fucopentaose I, Lacto-*N*-neofucopentaose I, Lacto-*N-*fucopentaose II, Lacto-*N*-fucopentaose III, Lacto-*N*-fucopentaose V, Lacto-*N-*neofucopentaose V, Lacto-*N*-difucohexaose I, Lacto-*N*-difucosylhexaose II, *para-*Lacto-*N*-fucosylhexaose, Fucosyl-lacto-*N*-sialylpentaose b, Fucosyl-lacto-*N-*sialylpentaose c, Fucosyl-lacto-*N*-sialylpentaose c, Disialyl-lacto-*N*-fucopentaose, 3-Fucosyl-3'-sialyllactose, 3-Fucosyl-6'-sialyllactose, Lacto-*N*-neodifucohexaose I, 3'-Sialyllactose, 6'-Sialyllactose, Sialyllacto-*N-*tetraosen LST-a, LST-b, LST-c, und Disialyllacto-N-tetraose.

## Revendications

1. Un procédé pour inhiber l'isomérisation d'un saccharide réducteur dans une solution aqueuse contenant ledit saccharide réducteur lors d'un traitement thermique pour stériliser ladite solution aqueuse de saccharide, le procédé comprenant l'étape d'acidification de la solution aqueuse de saccharide avant et/ou au cours du traitement thermique, où le traitement thermique comprend le chauffage de la solution aqueuse de saccharide à une température dans la gamme de 115°C à 150°C et le maintien de la température jusqu'à 60 minutes ; l'autoclavage de la solution aqueuse de saccharide à 121.1°C pendant 15 à 30 minutes ; le chauffage de la solution aqueuse à une température de 130°C à 150°C et le maintien de la température jusqu'à 5 minutes ; ou la soumission de la solution aqueuse à une température entre 71.5°C à 74°C, pendant 15 à 30 secondes, et où la solution aqueuse de saccharide est acidifiée en ajoutant au moins un acide à ladite solution aqueuse de saccharide.

2. Le procédé selon la revendication 1, où la solution aqueuse de saccharide est acidifiée à un pH ayant une valeur comprise entre 1 et 6.

3. Le procédé selon la revendication 1 ou 2, où l'acide est un acide inorganique choisi dans le groupe constitué par l'acide chlorhydrique, l'acide sulfurique, l'acide sulfureux, l'acide phosphorique, l'acide borique, l'acide fluorhydrique, l'acide bromhydrique, l'acide perchlorique, l'acide iodhydrique et l'acide carbonique.

4. Le procédé selon la revendication 1 ou 2, où l'acide est un acide organique choisi dans le groupe constitué par les acides monocarboxyliques, les acides dicarboxyliques et les acides tricarboxyliques.

5. Le procédé selon l'une des revendications 1 à 4, où le sucre réducteur est choisi dans le groupe constitué par les aldoses, les disaccharides et les oligosaccharides, où le disaccharide est choisi dans le groupe constitué par le lactose et le maltose.

6. Utilisation d'une solution aqueuse stérile de saccharide contenant au moins un saccharide réducteur obtenu par un procédé d'une des revendications 1 à 5, dans un procédé de production biotechnologique d'un oligosaccharide de lait maternel, le procédé comprenant les étapes consistant à :
- fournir au moins une cellule capable de produire l'oligosaccharide de lait maternel ;
- cultiver la ou les cellules dans un milieu de fermentation contenant la solution aqueuse stérile de saccharide et/ou étant complété par celle-ci, le saccharide réducteur étant présent dans la solution stérile à une concentration ≥0.66 M, pour que la ou les cellules produisent l'oligosaccharide de lait maternel ; et
- à purifier éventuellement le produit biologique issu du milieu de fermentation, où l'oligosaccharide de lait maternel est produit en une quantité ≥100 g/L dans le milieu de fermentation, en une quantité ≥150 g/L dans le milieu de fermentation, et en une quantité ≥200 g/L dans le milieu de fermentation à la fin du processus de fermentation.

7. L'utilisation selon la revendication 6, où le saccharide réducteur est présent dans la solution aqueuse stérile de saccharide à une concentration ≥ 1 M.

8. L'utilisation selon la revendication 6 ou 7, dans laquelle la solution aqueuse stérile est une solution aqueuse contenant du lactose stérilisée à la chaleur humide.

9. L'utilisation selon l'une des revendications 6 à 8, dans laquelle l'oligosaccharide de lait maternel est choisi dans le groupe composé de 2'-fucosyllactose, 3-fucosyllactose, 2',3-difucosyllactose, lacto-*N*-triose II, lacto-*N*-tétraose, lacto-*N-*néotétraose, lacto-*N*-fucopentaose I, lacto-*N*-néofucopentaose I, lacto-*N-*fucopentaose II, lacto-*N*-fucopentaose III, lacto-*N*-fucopentaose V, lacto-*N-*néofucopentaose V, lacto-*N*-difucohexaose I, lacto-*N*-difucosylhexaose II, *para-*Lacto-*N*-fucosylhexaose, fucosyl-lacto-*N*-sialylpentaose b, fucosyl-lacto-*N-*sialylpentaose c, fucosyl-lacto-*N*-sialylpentaose c, disialyl-lacto-*N*-fucopentaose, 3-fucosyl -3'-sialyllactose, 3-fucosyl-6'-sialyllactose, lacto-*N*-neodifucohexaose I, 3'-sialyllactose, 6'-sialyllactose, sialyllacto-*N*-tétraoses LST-a, LST-b, LST-c et disialyllacto-*N*-tétraose.
